# EUROPEAN PATENT SPECIFICATION

(11) **EP 3 993 865 B1**
(45) Date of publication and mention of the grant of the patent: **04.11.2026**
(21) Application number: 20834433.3
(22) Date of filing: 30.06.2020
(51) Int. Cl.: A61N 1/00, A61N 1/05, A61N 1/18, A61N 1/36, A61N 1/372

(54) **NEURAL BLOCK BY SUPER-THRESHOLD LOW FREQUENCY ELECTRICAL STIMULATION**
NEURONALER BLOCK DURCH NIEDRIGFREQUENTE ELEKTRISCHE STIMULATION MIT SUPERSCHWELLENWERT
BLOCAGE NEURONAL PAR UNE STIMULATION ÉLECTRIQUE À BASSE FRÉQUENCE SUPÉRIEURE AU SEUIL

(30) Priority: 03.07.2019 US 201962870230 P
(43) Date of publication of application: 11.05.2022
(60) Divisional application: 26208614.3
(73) Proprietor: University of Pittsburgh - Of the Commonwealth System of Higher Education, Pittsburgh, Pennsylvania 15260 (US)
(72) Inventor: TAI, Changfeng, Wexford, Pennsylvania 15090 (US)
(74) Representative: Kraus & Lederer PartGmbB
(86) International application number: PCT/US2020/040288
(87) International publication number: WO 2021/003151

(56) References cited:
- US-A1- 2002 193 840
- US-A1- 2005 015 117
- US-A1- 2007 073 354
- US-A1- 2008 058 888
- US-A1- 2010 036 454
- US-A1- 2010 036 454
- US-A1- 2012 101 326
- US-A1- 2013 035 740
- US-A1- 2013 035 740
- US-A1- 2015 134 028
- US-A1- 2017 050 024
- US-A1- 2017 182 319
- US-A1- 2018 344 518
- REITZ ANDR? ET AL: "Afferent fibers of the pudendal nerve modulate sympathetic neurons controlling the bladder neck", NEUROUROLOGY AND URODYNAMICS., vol. 22, no. 6, 1 January 2003 (2003-01-01), US, pages 597 - 601, XP093053278, ISSN: 0733-2467, DOI: 10.1002/nau.10134
- HANSEN J ET AL: "TREATMENT OF NEUROGENIC DETRUSOR OVERACTIVITY IN SPINAL CORD INJURED PATIENTS BY CONDITIONAL ELECTRICAL STIMULATION", JOURNAL OF UROLOGY, LIPPINCOTT WILLIAMS & WILKINS, BALTIMORE, MD, US, vol. 173, no. 6, 1 June 2005 (2005-06-01), pages 2035 - 2039, XP027855485, ISSN: 0022-5347, [retrieved on 20050601]
- KILGORE K L; BHADRA N: "Nerve conduction block utilising high-frequency altemating current", IN: MEDICAL AND BIOLOGICAL ENGINEERING AND COMPUTING, June 2004 (2004-06-01), XP019834123, Retrieved from the Internet <URL:https://www.researchgate.net/profile/Niloy_Bhadra/publication/8516425Nerve_conduction_block_utilising_high-frequency_alternating_current/links/55fc23dd08aec948c4b1066f/Nerve-conduction-block-utilising-high-frequency-a1ternating-current.pdf> [retrieved on 20200906], DOI: 10.1007/BF02344716

## Description

### BACKGROUND OF THE INVENTION

### Field of the Invention

The present teaching pertains to a method of nerve block and related devices, more specifically a method of blocking nerve conduction or neuron excitation by applying super-threshold electrical pulses to the nerve or neuron, and devices for carrying out such methods.

The present teaching also pertains to a method of treating urological or gastrointestinal disorders and related devices, more specifically a method of controlling micturition or defecation by applying super-threshold electrical pulses to the pudendal nerve or a branch thereof, and devices for carrying out such methods. The present invention is set out in the appended claims.

### Description of Related Art

Blocking nerve conduction or neuron excitation has a broad clinical application to treat many disorders including chronic pain, obesity, heart failure, bladder dysfunction or spasm after spinal cord injury, etc. However, currently electrical nerve block uses high kilohertz (e.g., 4 kHz or greater) electrical pulses in clinical applications, which requires use of a cuff electrode. Use of such electrodes requires invasive surgery to wrap the nerve or neuron with the cuff, limiting the clinical application of such methods.

Accordingly, there is a need in the art for a method of blocking nerve conduction or preventing neuron excitation to provide relief or otherwise treat a condition. Document US 2017/0182319 describes known devices and methods for blocking nerve conduction.

### SUMMARY

The present teaching provides a method of blocking a nerve or neuron in a patient, including: applying an electrical stimulation to the nerve or neuron, wherein the electrical stimulation is of an intensity that is greater than an excitation threshold of the nerve or neuron for a length of time sufficient to produce a block of nerve conduction or neuron excitation.

The present teaching provides also a device including: a controller; a pulse generator in communication with the controller; and an electrode, optionally one or more electrode leads, configured to be placed near or in contact with a nerve or neuron, the electrode being in electrical communication with the pulse generator, wherein the device is configured to apply an electrical stimulation to a nerve or neuron of a patient, wherein the electrical stimulation is of an intensity that is greater than an excitation threshold of the nerve or neuron for a length of time sufficient to produce a block of nerve conduction or neuron excitation.

The present teaching provides also a device including a controller; a pulse generator in communication with the controller; and one or more skin surface electrodes or electromagnetic coils in electrical communication with the pulse generator, wherein the pulse generator and one or more skin surface electrodes or electromagnetic coils are configured to apply an electrical stimulation to a nerve or neuron of a patient, wherein the electrical stimulation is of an intensity that is greater than an excitation threshold of the nerve or neuron for a length of time sufficient to produce a block of nerve conduction or neuron excitation.

The present invention is set out in the appended claims.

### BRIEF DESCRIPTION OF THE DRAWINGS

Additional advantages and details of the methods and devices according to the present teaching are explained in greater detail below with reference to the exemplary embodiments and aspects, and the following figures in which:

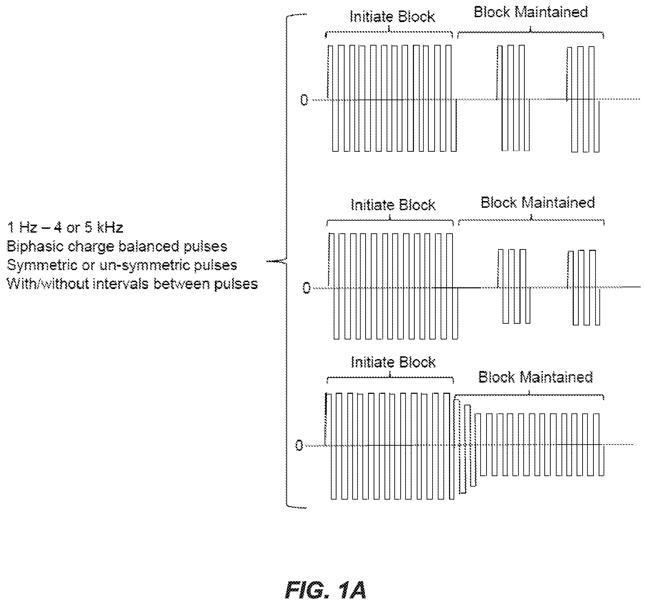

**FIGS. 1A-1B** show that once block is achieved with super-threshold stimulation at low frequency, it can be maintained in a variety of different manners/frequencies;

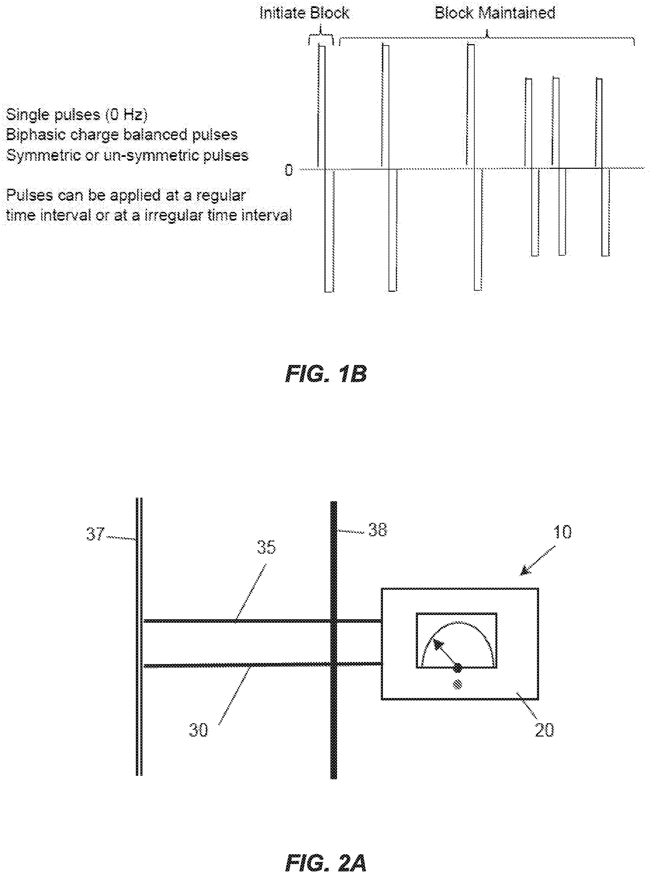

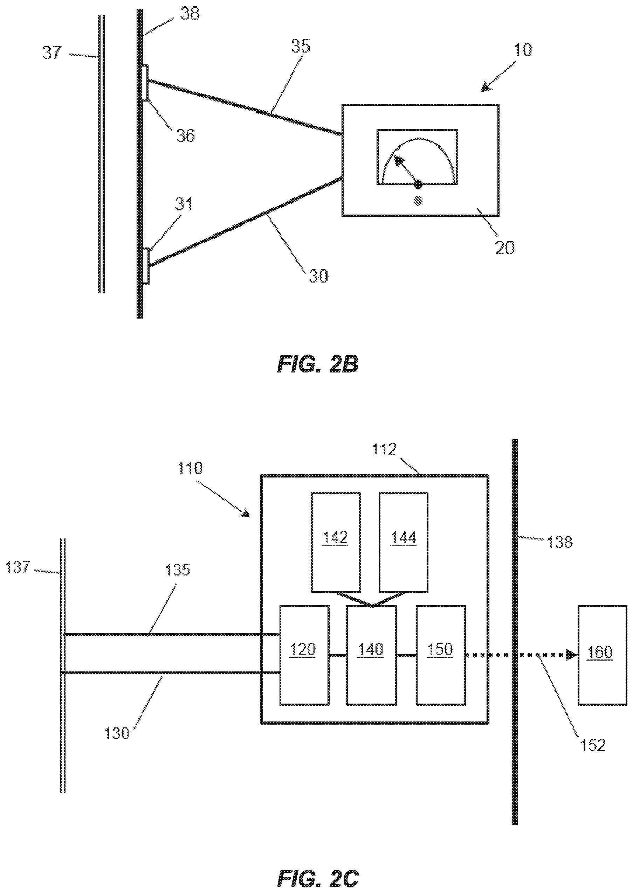

**FIGS. 2A-2C** are schematic diagrams of various external systems (Figures 2A and 2B), and implantable systems (Figure 2C) for use in blocking nerves as described herein;

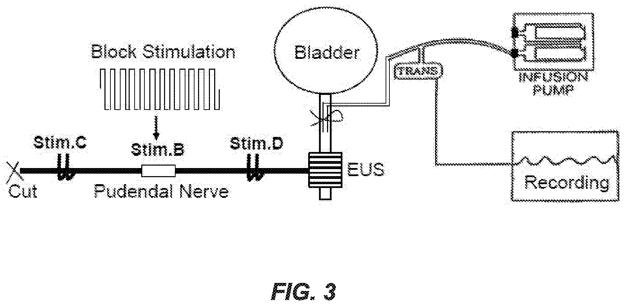

**FIG. 3** shows an experimental setup for application of blocking stimulation. Block stimulation is applied to the pudendal nerve by a tripolar cuff electrode (Stim.B) to block propagation of the action potentials induced by a bipolar hook electrode at a central site (Stim.C). Another bipolar hook electrode is placed at a distal site (Stim.D) to confirm that the external urethral sphincter (EUS) is not fatigued. The urethra is slowly perfused by an infusion pump so that the EUS contraction can be recorded by the increase in urethral pressure;

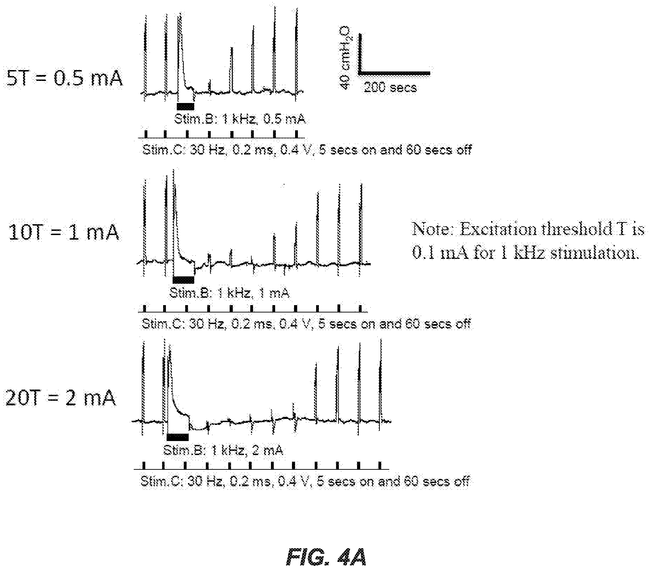

**FIGS. 4A-4B** show effect of different intensities on post-stimulation block delivered at 1 kHz, where excitation threshold T is 0.1 mA at 1 kHz;

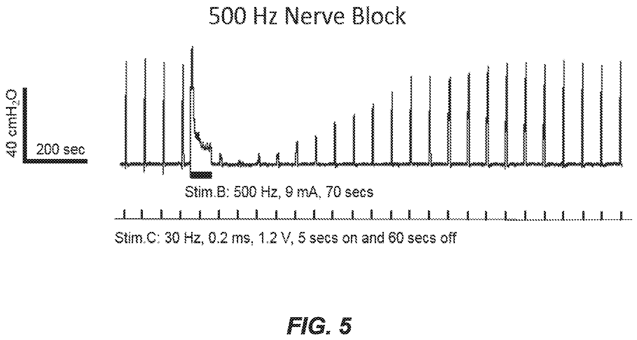

**FIG. 5** shows the post-stimulation blocking effect of stimulation delivered at 500 Hz, intensity of 9 mA, for 70 seconds;

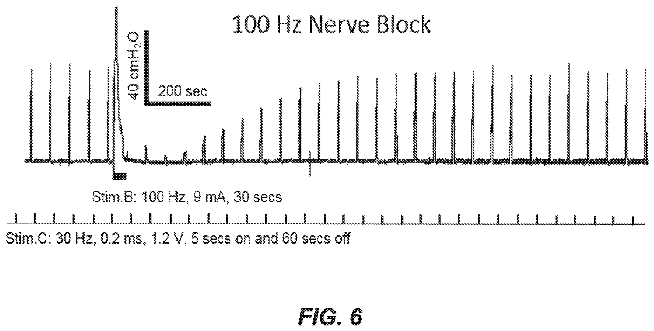

**FIG. 6** shows the post-stimulation blocking effect of stimulation delivered at 100 Hz, intensity of 9 mA, for 30 seconds;

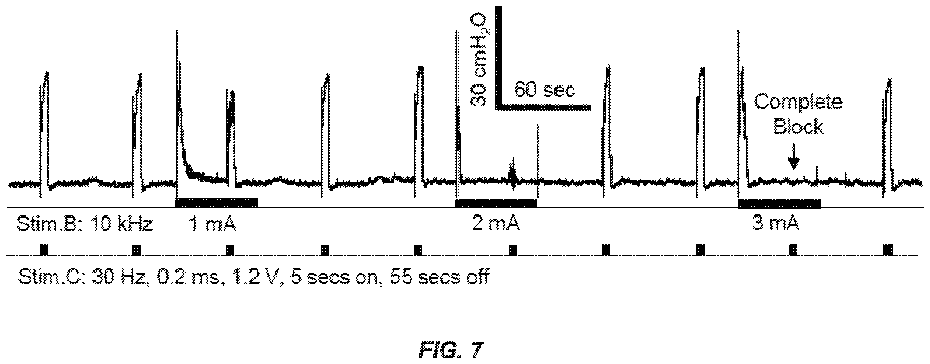

**FIG. 7** shows determination of the block threshold (T) as the stimulus intensity at which 10 kHz stimulation (Stim.B) completely blocked the EUS contraction induced by the 30 Hz intermittent (5 secs on, 55 secs off) stimulation at the central site (Stim.C). T = 3 mA as shown in this figure. The thick black bars under the pressure trace indicate the durations of stimulation. Please note that the 10 kHz Stim.B did not induce a post-stimulation block. A much longer stimulation duration (10-30 minutes) was required for 10 kHz to induce a post-stimulation block;

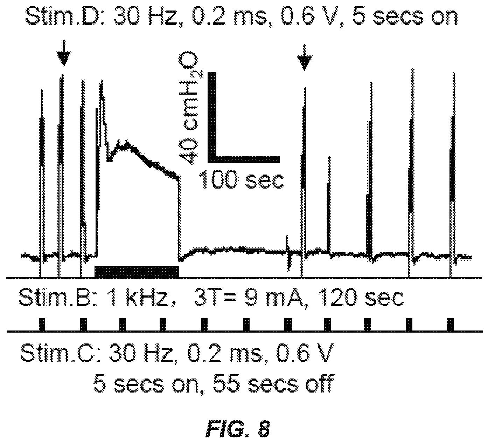

**FIG. 8** shows complete nerve block occurs after the end of the 1 kHz stimulation. The arrows indicate the EUS contractions induced by the 30 Hz stimulation at the distal site (Stim.D). The thick bars under the EUS pressure trace indicate the stimulation durations for Stim.B and Stim.C. After ending the 1 kHz Stim.B, EUS contraction can still be induced by the distal Stim.D but the contractions induced by the central Stim.C are completely blocked initially and then gradually recover, indicating that nerve conduction block occurs locally at Stim.B and the EUS is not fatigued;

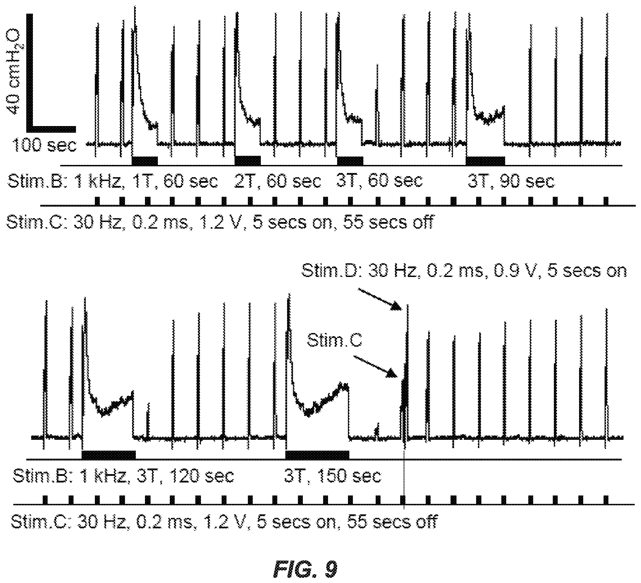

**FIG. 9** shows a protocol to determine the minimal intensity and duration for 1 kHz stimulation to induce a post-stimulation block that can reduce EUS contractions to <5 cm H₂O urethral pressure, i.e., a complete post-stimulation block. The bottom trace is the continuation of the top trace. The 2 arrows indicate the EUS contractions induced by the 30 Hz stimulation at the distal site (Stim.D) and the central site (Stim.C). The thick bars under the EUS pressure trace indicate the stimulation durations for Stim.B and Stim.C. T = 3 mA;

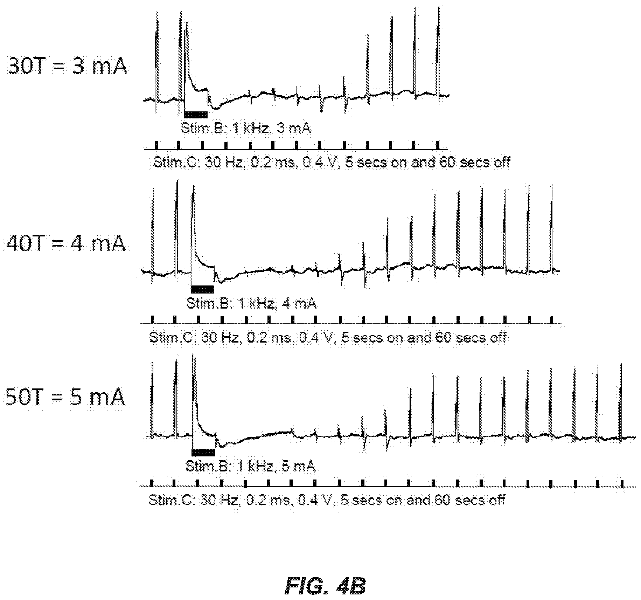

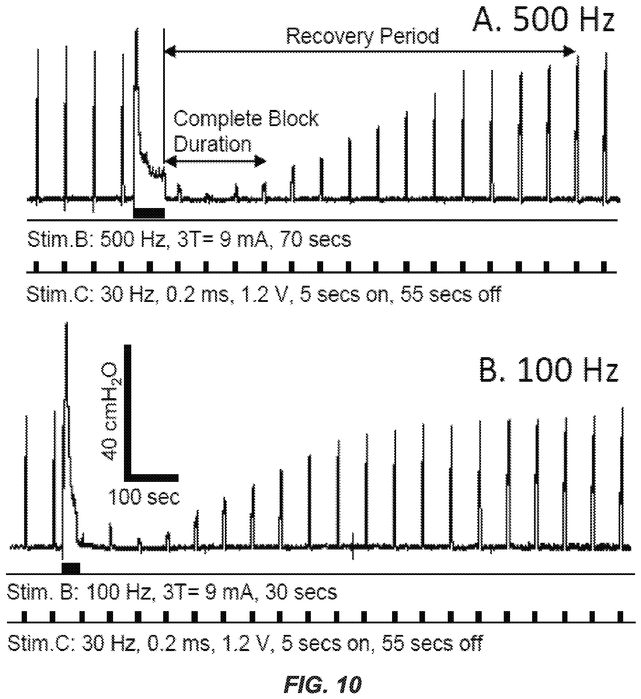

**FIG. 10** shows the minimal stimulation intensity and duration to induce a complete post-stimulation were also determined for 500 Hz (A) or 100 Hz (B) stimulation. The thick bars under the EUS pressure trace indicate the stimulation durations for Stim.B and Stim.C. The data were obtained in the same experiment as shown in FIG. 4. The recovery period is defined as the time period required for the EUS contraction pressure to reach >90% of the pre-stimulation pressure. The complete block duration is defined as the post-stimulation period during which Stim.C can only induce <5 cm H₂O urethral pressure;

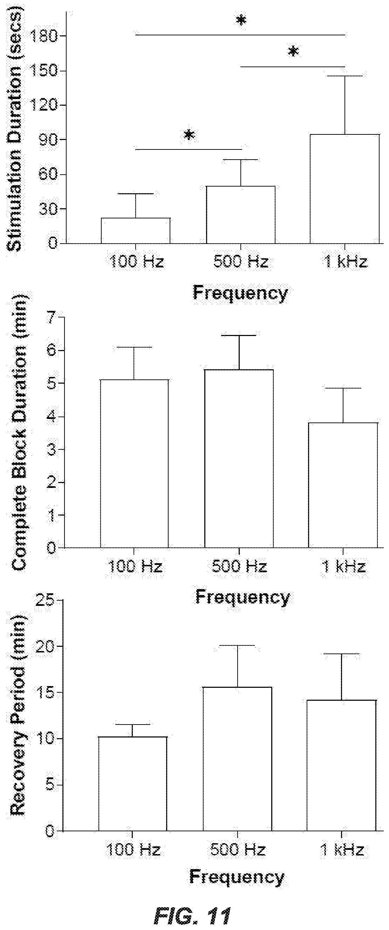

**FIG. 11** shows effects of stimulation frequency on the complete post-low frequency blocking stimulation (LFBS) block induced by LFBS at the minimal intensity and duration. A longer stimulation duration is required for higher frequencies to induce a complete post-LFBS block (A). However, complete block duration (B) and recovery period (C) do not change with stimulation frequency (p>0.05, un-paired t-test). The nerve block can occur at intensities of 1-3 T (3-9 mA) at different frequencies. N = 6 for 100 Hz, N = 9 for 500 Hz, and N = 14 for 1 kHz. * indicates significant different (p<0.05, unpaired t-test); and

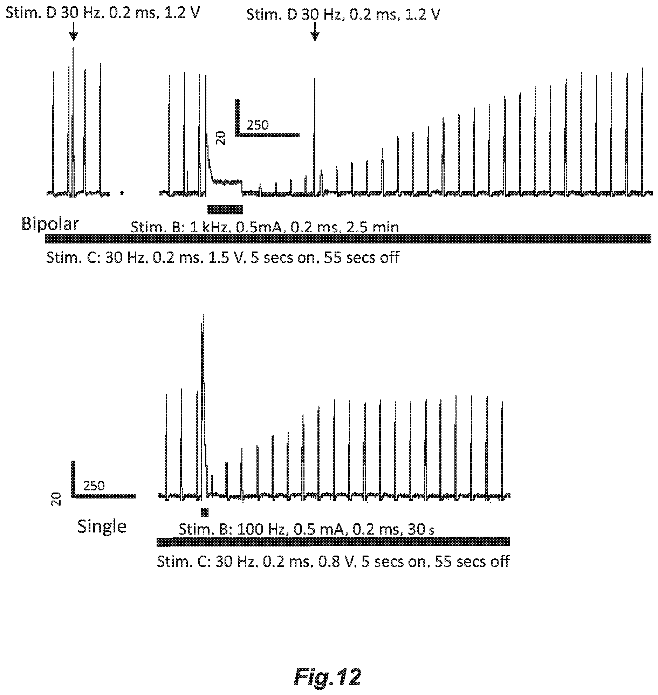

**FIG. 12** shows that use of a bipolar hook electrode (top panel) or a single electrode lead (bottom panel) can provide a LFBS and produce post-stimulation block.

### DESCRIPTION OF THE INVENTION

As used herein, the terms "communication" and "communicate" refer to the receipt, transmission, or transfer of one or more signals, messages, commands, or other type of data. For one unit or device to be in communication with another unit or device means that the one unit or device is able to receive data from and/or transmit data to the other unit or device. A communication can use a direct or indirect connection, and can be wired and/or wireless in nature. Additionally, two units or devices can be in communication with each other even though the data transmitted can be modified, processed, routed, etc., between the first and second unit or device. For example, a first unit can be in communication with a second unit even though the first unit passively receives data and does not actively transmit data to the second unit. As another example, a first unit can be in communication with a second unit if an intermediary unit processes data from one unit and transmits processed data to the second unit. It will be appreciated that numerous other arrangements are possible. Any known electronic communication protocols and/or algorithms can be used such as, for example, TCP/IP (including HTTP and other protocols), WLAN (including 802.11a/b/g/n and other radio frequency-based protocols and methods), analog transmissions, Global System for Mobile Communications (GSM), 3G/4G/LTE, BLUETOOTH, ZigBee, EnOcean, TransferJet, Wireless USB, and the like known to those of skill in the art.

As used herein, "electrical communication," for example in the context of transmitting electrical pulses from a pulse generator to an electrode refers to sending an electrical pulse produced by a pulse generator to a skin surface electrode, an electrode lead, a magnetic coil, or like devices capable of generating electrical current to stimulate a nerve or neuron as described herein, typically through an electrically-conductive lead, such as a wire.

As used herein, the "excitation threshold" (T) of a nerve or neuron is the minimum level to which a neuron and/or nerve membrane must be depolarized to initiate an action potential, resulting in excitation of the nerve or neuron, e.g., initiation of an action potential and propagation of the action potential, and thereby propagation of a signal in the nerve or neuron. The terms "nerve" and "neuron" are used interchangeably herein, particularly with reference to excitation thresholds, though one of skill in the art will appreciate that neuron refers to the cell body at which an action potential is generated and nerve refers to the axon along which an action potential is conducted. Stimulation parameters sufficient to excite a neuron will be considered suitable to excite or propagate an action potential in a nerve. Depolarization of a nerve or neuron membrane potential results in an increase in the membrane voltage, for example from -70 millivolts (mV) to up to +40 mV. The excitation threshold may depend on the frequency at which the stimulation is delivered. For example, 0.1 mA delivered at 1 kHz may depolarize a neuron to a point at or above the neuron's excitation threshold, while the same intensity delivered at 10 Hz may not depolarize the neuron to the same degree.

As used herein, the term "super-threshold depolarization" or "super-threshold stimulation" means a stimulation sufficient to increase membrane voltage of a nerve or neuron from resting membrane potential (e.g., -70mV) to a level at or above the excitation threshold, such that the nerve or neuron becomes excited, e.g., such that an action potential is initiated or conducted. It is noted that in the same nerve trunk, the motor and sensory nerve fibers may have different excitation thresholds; however, a membrane excitation threshold can be in the range of from -55mV to -45 mV, all subranges therebetween inclusive. For the same sensory nerve, the excitation thresholds for inducing paresthesia or pain are also different. Therefore, super-threshold as used herein means that stimulation intensity is at or above the level to induce muscle contraction, paresthesia, or pain. Super-threshold stimulation as described herein can increase the membrane voltage from resting (-70 mV) to a voltage equal to or greater than -55 mV.

The "intensity" of an electrical pulse is proportional to, and refers to either the voltage or current (e.g., milliAmperes or mA) applied to the nerve or neuron, with an increased intensity being proportional to an increased voltage or an increased current applied to the nerve or neuron. Intensity may be measured as, or proportional to, electrical power, e.g., Watts.

The present teaching provides a method of blocking a nerve or neuron, by applying a super-threshold electrical stimulation to the nerve or neuron. An intensity of the electrical stimulation that is applied to the nerve or neuron is greater than an excitation threshold of the nerve or neuron (e.g., depolarizing membrane voltage to equal to or greater than about - 55mV), such that an action potential is generated, and is referred to herein as "super-threshold" stimulation. This super-threshold stimulation is applied to the nerve or neuron for a length of time sufficient to produce a block of nerve conduction or neuronal excitation. The block induced by the super-threshold electrical stimulation can include a post-stimulation block.

As used herein, "post-stimulation block" refers to a nerve block that extends past the cessation of the electrical stimulation, and can, depending on the duration and intensity of the electrical stimulation, persist from seconds to hours, days, weeks, months, or years, including increments therebetween. The post-stimulation block can last at least 1 minute. The post-stimulation block can be maintained after a cessation of stimulation for at least 1 minute, optionally at least 5 minutes, 10 minutes, 15 minutes, or 30 minutes, at which time stimulation can be re-applied. The stimulation that is re-applied can be the same or different intensity and frequency as compared to the initial parameters used to initiate the block. This may be due to movement of ions and the possibility of an increase in excitation threshold achieved through super-threshold stimulation, which, without wishing to be bound by the theory, occurs by the reallocation of sodium and potassium during the application of super-threshold stimulation. After post-stimulation block is achieved, the frequency and/or intensity of the electrical stimulation can be altered to maintain or re-initiate the block. That is, after achieving post-stimulation block, the frequency of the stimulation can be increased or decreased, and/or the intensity of the stimulation can be increased or decreased.

The super-threshold stimulation can include electrical pulses (including magnetic stimulation capable of generating an electrical current) that can have any suitable characteristic, so long as the stimulation is super-threshold stimulation. As such, the terms "electrical stimulation" and "electrical pulses" are used interchangeably herein. As will be recognized by a person of skill in the art, characteristics of the electrical pulses, including, without limitation, amplitude (magnitude or size of a signal voltage or current), voltage, amperage, duration, frequency, polarity, phase, relative timing and symmetry of positive and negative pulses in biphasic stimulation, and/or wave shape (e.g., square, sine, triangle, sawtooth, or variations or combinations thereof) may be varied in order to provide a desired super-threshold stimulation and resultant post-stimulation blocking in a patient or class of patients. So long as other characteristics of the electrical signals (e.g., without limitation, amplitude, voltage, amperage, duration, polarity, phase, relative timing and symmetry of positive and negative pulses in biphasic stimulation, and/or wave shape) are within useful ranges, modulation of the pulse frequency will achieve the desired result of super-threshold induced blocking of a nerve or neuron.

Turning to FIGS. 1A and 1B, these figures show non-limiting examples of how to initiate super-threshold nerve block and then maintain the block for a long period of time. Nerve block can be initiated by super-threshold stimulation of a certain duration, and the block will persist after ending the stimulation (e.g., post-stimulation block). The post-stimulation block period can be extended by applying additional stimulation after the initiation of the block. The additional stimulation can be of the same or reduced intensity and applied either intermittently or continuously, or at a different frequency **(****FIG. 1A****).** Furthermore, the post-stimulation block can also be initiated by single or multiple stimulation pulses at an extreme super-threshold intensity and be maintained by multiple single pulses at a reduced intensity applied at regular or irregular time points **(****FIG. 1B****).**

One characteristic of the electrical signals used to produce a desired response, as described above, is the frequency of the electrical pulse. Although effective frequency ranges (e.g., frequencies able to produce a stated effect) may vary subject-to-subject, and the controlling factor is achieving a desired outcome, certain, non-limiting exemplary ranges may be as follows, with the proviso that the stimulation, or pulses, evoke an action potential in the target nerve/neuron. For blocking nerves, useful frequencies may range above 1 Hz (Hertz), from approximately 1 Hz to less than 4 kHz (kilohertz), or from 1 Hz to 1.5 kHz, or from 1 Hz to 1.3 kHz, or from 100 Hz to 1 kHz, optionally from 100 Hz to 500 Hz, all subranges therebetween inclusive. Effective stimulation may be delivered at 100 Hz, 500 Hz, 1 kHz, 1.5 kHz, or any value therebetween, or in a range of 1 Hz to less than 4 kHz, 1 Hz to 1.3 kHz, 1 Hz to 1.5 kHz, 100 Hz to 1 kHz, or 100 Hz to 500 Hz, all subranges therebetween inclusive. While nerve block obtained with super-threshold high frequency stimulation (≥ 4 kHz) is known, it was heretofore unknown that super-threshold stimulation in the low kilohertz range (e.g., < 4 kHz) or below kilohertz range (1-1000 Hz) could induce nerve block.

As indicated above, super-threshold electrical pulses are determined by the intensity of electrical stimulation, which in a medium of stable or relatively stable resistance, such as mammalian tissue, can be characterized as relating to current (*I*, typically measured in mA), or voltage (*V*, typically measured in mV), based on Ohm's Law. It should, therefore, be understood that the intensity of the stimulation is a matter of both *V* and *I*, and as such, both are increased, e.g., proportionally or substantially proportionally, with increased intensity of stimulation. As such, one characteristic of the pulses is the current that is applied to produce a super-threshold stimulation that is capable of nerve blocking. Super-threshold stimulation can be achieved in a typical range of from 0.1 mA to 100 mA, from 0.5 mA to 50 mA, from 0.5 mA to 5 mA, all subranges therebetween inclusive. Stimulation can be applied at 0.1 mA, 0.5 mA, 1 mA, 2 mA, 3 mA, 4 mA, 5mA, 10 mA, 50 mA, 100 mA, or any value therebetween. Super-threshold nerve stimulation can be achieved in a typical range of from 1 mV to 500 V.

Breaks, or periods where no electrical stimulation is applied, or is applied less frequently than necessary to achieve the post-stimulation block, can be introduced. When breaks or periods where no electrical stimulation are applied, the breaks or periods can be at least 10 seconds, optionally at least 1 minute, optionally at least 5 minutes, 10 minutes, 15 minutes, 30 minutes, or 180 minutes, wherein the block of nerve conduction or neuron excitation is maintained during the breaks or periods. Stimulation can then be reintroduced to maintain blockage. This reintroduced stimulation can be the same as originally applied, or of an increased or reduced intensity and/or an increased or reduced frequency compared to the stimulation providing the initial block.

Super-threshold stimulation can be measured in terms of excitation threshold (T) of a nerve or neuron, which allows for use of a metric that can be applied across patients no matter the individual variations in current and/or voltage necessary to induce excitation in a given patient. For example, and without limitation, reference can be made to excitation of 5T, or five-times the excitation threshold for a nerve of interest in a given patient, rather than to a specific combination of current/voltage. The stimulation that can be applied to a nerve in a patient to generate nerve block is 1T, 2T, 3T, 4T, 5T, 10T, 20T, 30T, 40T, 50T, 60T, 70T, 80T, 90T, 100T, or higher, all values therebetween inclusive.

As described below, the waveform of the pulses may vary, so long as the desired super-threshold blocking effect is realized. One skilled in the art will appreciate that other types of electrical stimulation may also be used in accordance with the present teaching. Monophasic or biphasic stimuli, or a mixture thereof, may be used. Damage to nerves by the application of an electrical current may be minimized, as is known in the art, by application of biphasic pulses or biphasic waveforms to the nerve(s), as opposed to monophasic pulses or waveforms that can damage nerves in some instances of long-term use. "Biphasic current," "biphasic pulses," or "biphasic waveforms" refer to two or more pulses that are of opposite polarity that may be of equal or substantially equal net charge (hence, biphasic and charge balanced), and may be symmetrical, asymmetrical, or substantially symmetrical. This is accomplished, for example, by applying through an electrode one or more positive pulses, followed by one or more negative pulses, typically of the same amplitude and duration as the positive pulses, or vice versa, such that the net charge applied to the target of the electrode is zero, or approximately zero. For charge-balanced biphasic stimulation, the opposite polarity pulses may have different amplitudes, profiles, or durations, so long as the net applied charge by the biphasic pulse pair (the combination of the positive and negative pulses) is approximately zero.

The waveform may be of any useful shape, including without limitation: sine, square, rectangular, triangular, sawtooth, rectilinear, pulse, exponential, truncated exponential, or damped sinusoidal. The pulses may increase or decrease over the stimulation period. The waveform can be rectangular. The super-threshold pulses may be applied continuously or intermittently as needed. As indicated below, super-threshold stimulation of a nerve or neuron at certain voltages or currents for certain time periods elicits post-stimulation nerve blockage. Therefore, the super-threshold stimulation may be applied for very short intervals (e.g., 5 seconds to 70 seconds), short intervals (e.g., 1-10 minutes), or longer intervals (e.g., 30 minutes, 360 minutes or even longer, for example days, weeks, months, or even years) to achieve shorter-lasting blockage/relief in terms of at least 10 seconds, 30 seconds, 1 minute, 2 minutes, 3 minutes, 5 minutes, 10 minutes, or 180 minutes, or achieve longer-lasting blockage/relief, in terms of hours, days, weeks, months, or years. The stimulation can be applied for at least 5 seconds, 30 seconds, at least 70 seconds, or at least 5 minutes. The stimulation can be applied for 30 minutes to 2 hours, all subranges therebetween inclusive. The stimulation can be applied for at least 70 minutes, at least 80 minutes, or at least 90 minutes. The stimulation can be applied intermittently (that is, the pulses are turned on and off alternately during a stimulation interval for any time period) during continuous or interval stimulation protocols. For example, the stimulation may be applied for 5 seconds on and 5 seconds off over an interval of, for example, 1-10 minutes or longer (e.g., hours, days, weeks, months, years). Other examples of intermittent application of pulses may be 1-90 seconds on and 1-90 seconds off over up to a 360 minute time period or longer. So long as other pulse parameters (e.g., intensity and frequency) are within acceptable limits (e.g., those known to not to cause damage and/or long-term injury to a neuron or nerve), the inhibition is temporary and does not damage the involved neurons/nerves. For example, intermittent application of pulses may be continuous, that is, for as long as the pulses are having the desired effect, and for as long as the patient desires (e.g., is not undesirably painful, or harmful to the patient). In one aspect, the stimulation is provided continuously, for example, to treat severe symptoms, or any symptom that does not respond to intermittent, short-term stimulation to the degree desired by a clinician or the patient.

Also provided herein is a method of controlling a physiological process, such as a urological or gastrointestinal process, by applying super-threshold electrical stimulation to a patient to block nerve conduction. The physiological process can be micturition. The physiological process can also be defecation. U.S. Patent No. 8,805,510, incorporated herein by reference in its entirety, describes physiological processes that can be modulated and/or controlled by administration of electrical stimulation as described herein, including urinary incontinence, overactive bladder, urine retention and voiding dysfunction, detrusor sphincter dyssynergia, fecal incontinence, constipation, irritable bowel syndrome, sexual dysfunction in both men and women, premature ejaculation, decreased sexual sensation, an orgasm, urethral pain, prostate pain, vulvodynia, anal pain, rectal pain, and bladder pain. Such conditions can result from neurological impairment or from other diseases or conditions. For example, urinary retention and/or incontinence can result from spinal cord injury or stroke, or damage caused by trauma, disease (e.g., multiple sclerosis) and/or congenital defects. Circumstances where one or more of the conditions is caused by spinal cord trauma, or other injury that reduces and/or eliminates sensation, such as pain sensation, may benefit from the methods disclosed herein, as the patient will be less likely to experience pain from the initial excitation that accompanies the super-threshold stimulation-induced block.

The method can include the step of applying super-threshold electrical stimulation to a patient's pudendal nerve or a branch thereof. The pudendal nerve originates in the sacral plexus and derives its fibers from the first, second, third, and fourth sacral nerves (S1, S2, S3, S4). The pudendal nerve passes between the piriformis and coccygeus muscles and leaves the pelvis through the lower part of the greater sciatic foramen. The nerve then crosses the spine of the ischium, and reenters the pelvis through the lesser sciatic foramen. The pudendal nerve accompanies the internal pudendal vessels upward and forward along the lateral wall of the ischiorectal fossa, and is contained in a sheath of the obturator fascia termed the pudendal canal. The pudendal nerve gives off the inferior rectal nerves. It then divides into two terminal branches: the perineal nerve, and the dorsal nerve of the penis (males) or the dorsal nerve of the clitoris (in females). The inferior anal nerves branch off shortly after passing through the greater sciatic foramen. The dorsal nerve of the penis or dorsal nerve of the clitoris are the more superficial terminal branch of the pudendal nerve while the perineal nerve is deeper terminal branches of the pudendal nerve, traveling into the deep perineal pouch.

The pudendal nerve carries both sensory (afferent) and motor (efferent) signals. It innervates, among other things, the anal and external urethral sphincters. It also innervates the penis and clitoris, bulbospongiosus and ischiocavernosus muscles, and areas around the scrotum, perineum, and anus. At sexual climax, peristaltic action of muscles in the reproductive ducts and accessory glands (e.g., seminal vesicles, prostate and Cowper's (bulbourethral) glands), along with spasms in the bulbospongiosus and ischiocavernous muscles result in ejaculation in the male. Spasms in the bulbospongiosus and ischiocavernous muscles accompany most of the feelings of orgasm in both sexes.

The method can be used to control micturition. Micturition, also called voiding or urination, is the act of emptying the bladder. In humans, when about 200 ml of urine has accumulated, distension of the bladder wall typically activates stretch receptors, triggering a visceral reflex arc. Afferent impulses are transmitted to the sacral region of the spinal cord, and efferent impulses return to the bladder via the parasympathetic pelvic nerves, causing the detrusor muscle of the bladder to contract and the internal sphincter of the bladder to relax. As the contractions increase in intensity, they force stored urine through the internal sphincter into the upper part of the urethra. Afferent impulses are also transmitted to the brain, so one feels the urge to void at this point. Because the external urethral (urinary) sphincter is voluntarily controlled, a person can choose to keep it closed and postpone bladder emptying temporarily. On the other hand, if the time is convenient, the voluntary sphincter can be relaxed, allowing urine to be expelled from the bladder. When one chooses not to void, reflex bladder contractions subside within a minute or so and urine continues to accumulate. After 200-300 ml more has collected, the micturition reflex occurs again and, if urination is delayed again, is damped once more.

Thus, normal bladder activity is typically divided into two phases. In the first phase, the "storage phase," the bladder detrusor is quiet and the external urethral sphincter (EUS) is closed. In the second phase, the "voiding phase," the bladder detrusor contracts and the EUS is (voluntarily) relaxed, permitting urine to flow out of the urethra. In patients with neurological damage affecting the micturition process, this process is disrupted, leading to, for example, incontinence or retention.

Incontinence is the inability to control micturition. Incontinence typically is a result of emotional problems, physical pressure during pregnancy, or nervous system problems, such as stroke or spinal cord lesions.

In urinary retention, the bladder is unable to expel its contained urine. Urinary retention is common after general anesthesia has been given (it seems that it takes a little time for the smooth muscles to regain their activity). Urinary retention in men often reflects prostate hypertrophy, narrowing the urethra, making it difficult to void. Stretching of the bladder wall by urine causes sensory impulses to be transmitted to the sacral region of the spinal cord. Motor impulses are delivered to the bladder detrusor muscle and the internal sphincter via parasympathetic fibers of the pelvic nerves. The pudendal nerve serves the striated muscle fibers of the external urethral sphincter.

Defecation proceeds by a similar manner as micturition. Sensory and motor control of defecation travels through the pudendal nerve. The rectum usually is empty. When feces are forced into the rectum by mass movement, the rectal wall is stretched, initiating the defecation reflex. In the defecation reflex, the walls of the sigmoid colon and rectum contracts and the anal sphincters relax, forcing the feces into the anal canal. The brain, however, decides whether the passage of feces should be temporarily stopped. If they are stopped, the rectal walls relax, until another mass-movement initiates another defecation reflex.

Patients with supra-sacral spinal cord injuries typically have no voluntary control over the micturition, defecation and ejaculatory processes. For example, after spinal cord injury (SCI) incontinence occurs frequently due to detrusor overactivity. Meanwhile, the bladder also does not empty well due to detrusor sphincter dyssynergia (DSD) resulting in a large residual volume of urine. Thus, the management of bladder function after SCI is a challenging task, because it requires inhibition of detrusor overactivity during urine storage and induction of a large amplitude bladder contraction at the same time relaxation of the EUS to empty the bladder. Current treatment for bladder dysfunction after SCI has either limited success or requires major invasive spinal surgery to implant stimulating electrodes on spinal roots. Intermittent urethral catheterization is the most common method for managing urinary tract dysfunction. However, it can lead to frequent bladder infections. Methods and devices disclosed herein may be of particular use in individuals with SCI, as the methods can be utilized to assist in control of micturition and defecation, without the concern that the super-threshold stimulation would result in undesirable pain.

Also provided herein is a method of controlling micturition and/or defecation. The method includes applying a first electrical stimulation to the left pudendal nerve or branches thereof and a second electrical stimulation to the right pudendal nerve or branches thereof of a patient. The first and second electrical stimulation can be of an intensity that is greater than an excitation threshold of the pudendal nerve, and can include any parameters and characteristics disclosed herein as being suitable for providing nerve block by LFBS. The stimulation can be applied for a length of time sufficient to produce a conduction block of both pudendal nerves either during or after ending the first and/or second electrical stimulation, thereby inhibiting contraction of the external urethral sphincter (EUS) and/or external anal sphincter (EAS).

Following the block, or during the blocking stimulation, when the EUS/EAS is relaxed, a third electrical stimulation to the left or right pudendal nerve can be applied at a location central to the blocked pudendal nerve site to induce bladder or colon/rectal contraction. Alternatively, again either during or immediately after ending the first and/or second electrical stimulation when the EUS/EAS is relaxed, a third electrical stimulation can be applied to a sacral spinal root (S1, S2, S3, or S4) to induce bladder or colon/rectal contraction. Alternatively, again either during or immediately after ending the first and/or second electrical stimulation when the EUS/EAS is relaxed, a third electrical stimulation can be applied to the spinal cord by epidural electrodes, or skin surface electrodes or electromagnetic coils to induce bladder or colon/rectal contraction. Alternatively, again either during or immediately after ending the first and/or second electrical stimulation when the EUS/EAS is relaxed, applying a third electrical stimulation to pelvic nerve to induce bladder or colon/rectal contraction. Alternatively, and again either during or immediately after ending the first and/or second electrical stimulation and during the EUS/EAS relaxation, pressure can be applied to the abdominal area to produce bladder or colon/rectal pressure. With regard to the steps involving stimulation, devices as described herein (discussed below) can include memory having programming instructions stored thereon, the programming instructions causing a processor to perform (through control of, e.g., a pulse generator) the various stimulations described above.

Also disclosed herein is a method of controlling micturition and/or defecation in a patient by applying first electrical stimulation to either left or right pudendal nerve or branches thereof of a subject, wherein the electrical stimulation is of an intensity that is greater than an excitation threshold of the pudendal nerve for a length of time sufficient to produce a block of pudendal nerve conduction either during or after ending the electrical stimulation, thereby inhibiting contraction of the external urethral sphincter (EUS) and/or external anal sphincter (EAS). The method further includes, either during or immediately after ending the first electrical stimulation when the EUS/EAS is relaxed, applying a second electrical stimulation to the same side of the blocked pudendal nerve at a location central to the blocked pudendal nerve site to induce bladder or colon/rectal contraction. Alternatively, and either during or immediately after ending the first electrical stimulation when the EUS/EAS is relaxed, a second electrical stimulation can be applied to a sacral spinal root (S1, S2, S3, or S4) to induce bladder or colon/rectal contraction. Alternatively, and again either during or immediately after ending the first electrical stimulation when the EUS/EAS is relaxed, a second electrical stimulation can be applied to the spinal cord by epidural electrodes, or skin surface electrodes or electromagnetic coils to induce bladder or colon/rectal contraction. Alternatively, and again either during or immediately after ending the first electrical stimulation when the EUS/EAS is relaxed, a second electrical stimulation can be applied to the pelvic nerve to induce bladder or colon/rectal contraction. Alternatively, and again either during or immediately after ending the electrical stimulation when the EUS/EAS is relaxed, pressure can be applied to the abdominal area to produce bladder or colon/rectal pressure. With regard to the steps involving stimulation, devices as described herein (discussed below) can include memory having programming instructions stored thereon, the programming instructions causing a processor to perform (through control of, e.g., a pulse generator) the various stimulations described above.

While urological and gastrointestinal processes are exemplified above, those of skill in the art will appreciate that the methods and devices described herein can be useful for treating other systems/processes where blockage of nerve conduction may prevent, improve, resolve, or reverse a condition or disease. For example, and without limitation, blockage of conduction of afferent pain fibers, blockage of the splanchnic nerve for treating heart failure (*see* Fidum et al., Splanchnic nerve block for acute heart failure, Circulation, 2018, Vol. 138, pp. 951-53) and blockage of conduction of the vagus nerve for treating obesity (*see* Sarr et al., The EMPOWER Study: Randomized, prospective, double-blind, multicenter trial of vagal blockage to induce weight loss in morbid obesity, Obes Surg, 2012) can be achieved with the devices and methods disclosed herein.

Also provided herein are devices and systems for applying super-threshold stimulation in a manner sufficient to induce post-stimulation nerve/neuron block. **FIG. 2A** provides a general schematic of one non-limiting embodiment or aspect of an electrical stimulation device **10** useful in the methods described herein. The device **10** includes a power supply or pulse generator **20.** The power supply/pulse generator **20** may be fixed output, or may be adjustable, for example within a useful range as described herein. The device **10** includes a first conductive lead **30** and, optionally, a second conductive lead **35.** While **FIG. 2A** shows leads **30, 35** in contact with nerve **37,** those of skill in the art will appreciate that conductive leads need not be in such close proximity such that they contact the nerve or neuron of interest. For example, and without limitation, leads **(30 and/or 35)** can be placed within 10 mm, 9 mm, 8 mm, 7 mm, 6 mm, 5mm, 4mm, 3mm, 2mm, or 1mm of a nerve or neuron of interest, all subranges therebetween inclusive.

A benefit of the present methods, and the use of super-threshold stimulation, is the option to utilize one or more leads that need not be cuffed (e.g., need not be placed on a nerve or neuron, but instead may be placed merely in proximity to the nerve or neuron of interest). Conductive leads **30, 35** can be directly wired to power supply/pulse generator **20,** or may each comprise multiple leads and electrical connectors, fasteners, terminals, or clips to produce a contiguous electrical connection between the power supply/pulse generator **20** and the end of the leads. One or more leads for grounding the circuit (not shown) can also be provided, and can be attached to the patient's body. Skin **38** is also shown, and as such the device **10** is external and can be a hand-held or body-worn device - held in place by a belt or strap, such as by a hook and loop fastener band, though optionally, the device **10** can be an implantable device (described in more detail below). In **FIG. 2A****,** the leads are of opposite polarity and, together, form a circuit for application of any electrical waveform described herein. Alternative designs, with different leads, probes, electrodes, or electrical contacts, or combinations thereof will be apparent to those of ordinary skill. As used herein, an "electrical contact" is inclusive of any structure useful for directly applying an electrical current to a nerve or tissue in a patient, such as to the skin of a patient. Structures for producing a magnetic field, and therefore an electrical current via induction, are not considered to be electrical contacts. Nevertheless, induction probes, that is structures capable of generating a magnetic field capable of producing an electrical current, can be used to produce the electrical pulses described herein.

**FIG. 2B** depicts schematically another aspect of a device **10** for nerve block, which, like the device of **FIG. 2A****,** has an external power supply. In **FIG. 2B****,** like reference numbers as compared to reference numbers **of** **FIG. 2A****,** refer to like elements of the device **10.** However, surface electrodes **31** and **36** are utilized, and stimulation is transcutaneous. In an alternative aspect, not shown, surface electrodes **31** and **36** are replaced by electromagnets for magnetic induction stimulation of impulses in nerve **37.**

**FIG. 2C** depicts a further aspect of the nerve block device **110** that is implanted, and includes an implantable housing **112.** The housing **112** contains various subunits of the device, including a power supply/pulse generator **120** connected to a first lead **130** and a second lead **135** for stimulating a nerve **137.** Skin **138** is depicted for context. The housing may be composed of any biocompatible material as are known in the medical fields for use in such implantable devices, such as a plastic, metal, carbon fiber, or ceramic material, or a polymer-coated material, such as a metal or plastic housing coated with a biocompatible polymer or hydrogel. The housing **112** also contains various connected subunits of the device **110,** including a processor **140,** a storage module **142** including transient data storage (e.g., RAM), and non-transient data storage, such as flash memory or a solid-state drive, and a battery 144 that is optionally rechargeable by electromagnetic induction. The processor **140** can also be connected to a wireless communications module **150** for communicating wirelessly, e.g., by near-field communication, or by BLUETOOTH, Wi-Fi, or over a cellular network, with an external computer or computer network, such as a smartphone, tablet, laptop, personal computer, smart watch, workstation, server, or computer network.

The devices of **FIGS. 2A-2C** can be battery-powered, and optionally the battery is rechargeable. Where the device is implanted, the device can be recharged by wireless, e.g., magnetic induction recharging methods, as are known. The devices of **FIG. 2A** **and/or** **FIG. 2B** also can include a communications interface, such as a wireless communications interface or module, for transmitting data, and for receiving instructions from a separate computing device, such as from a controller app or software on a smartphone, tablet, laptop, personal computer, workstation, server, or computer network. As would be appreciated by those of ordinary skill in the fields of computer and software engineering, a multitude of potential device and system configurations and implementation schemes can be used to control devices and systems that provide electrical stimulation and nerve block as described herein.

Referring to **FIG. 2C****,** but equally applicable to any aspect of the device, e.g., device **10** of **FIG. 2A** **and/or** **FIG. 2B****,** the device **110** comprises a controller for executing functions related to electrical pulse output of the power supply. In some examples, a controller is a central processing engine including a baseline processor, memory, and communications capabilities. For example, the controller can be any suitable processor comprising computer readable memory and configured to execute instructions either stored on the memory or received from other sources. Computer readable memory can be, for example, a disk drive, a solid-state drive, an optical drive, a tape drive, flash memory (e.g., a non-volatile computer storage chip), cartridge drive, and control elements for loading new software.

In some examples, the controller includes a program, code, a set of instructions, or some combination thereof, executable by the processor for independently or collectively instructing the device to interact and operate as programmed, referred to herein as "programming instructions". In some examples, the controller is configured to issue instructions to the power supply/pulse generator to initiate super-threshold electrical pulses, and to control output parameters of the power supply in a manner sufficient to induce nerve/neuron block, optionally post-stimulation block, as described throughout this disclosure (e.g., super-threshold stimulation, altering stimulation parameters once block has been achieved, and the like). Those of skill in the art will appreciate that a processor associated with a device **10, 110** disclosed herein can be programmed to deliver suitable super-threshold stimulation as described generally throughout this disclosure. In any case, the controller is configured to receive and process electrical pulse parameters, either programmed into the device or from an external source, and optionally to output data obtained from the power supply as feedback to determine if the power supply is producing a desired output. Processing can include applying filters and other techniques for removing signal artifacts, noise, baseline waveforms, or other items from captured signals to improve readability.

Further to the above, the device **10, 110** can include programming instructions that, when executed by the processor **140,** cause the power supply/pulse generator **120** to apply electrical stimulation at an intensity at or above an excitation threshold of the nerve/neuron (e.g., at or above -55 mV) for a time sufficient to cause a block and/or a post-stimulation block in the nerve or neuron, or to deliver a physiological outcome as described above. Useful parameters are described above, but can include stimulation at, for example and without limitation, 100 Hz, 500 Hz, 1 kHz, from 1 Hz to less than 4 kHz or from 1 Hz to 1.5 kHz, from 1 Hz to 1.3 kHz, from 100 Hz to 1.5 kHz, from 100 Hz to 500 Hz, from 500 Hz to 1.5 kHz, all values subranges therebetween inclusive, at an intensity of, for example and without limitation, 1 mA, 3 mA, 9mA, 12 mA, 15mA, or from 0.1 mA to 50 mA, for a duration of seconds to days, all subranges therebetween inclusive for all parameters.

As also described previously, the processor **140** can thereafter instruct the power source/pulse generator **120** to apply a first decreased or increased intensity and/or frequency electrical stimulation following initiation of block. The controller can be programmed or configured to, once block of nerve conduction or neuron excitation is achieved, instruct the pulse generator to change the intensity and/or frequency of the electrical stimulation. Various sensors and devices can be utilized to determine whether block has been achieved. For example, as described above and illustrated in the examples below, a device can include more than one contact or lead. One of the contacts/leads can be located proximally of the blocking contact/lead, and blocking can be determined by whether a stimulation pulse applied proximally of the block results in transmission of an action potential distally of the location of the blocking contact/lead.

A device **10, 110** can include multiple channels and multiple electrode leads/contacts, to provide a blocking stimulation and an excitatory stimulation, such that the device **10, 110** can, through application of stimuli at various times, locations, and intensities, induce micturition, defecation, and/or retention. Stimulation parameters and electrode lead/contact placement for inducing such physiological actions are disclosed in U.S. Patent No. 9,623,243, the contents of which are incorporated herein by reference in their entirety. Briefly however, for example and without limitation a device **10, 110** as described herein, whether implanted, wearable, or otherwise disposed externally, can include multiple channels, one (or more) channels for providing a blocking stimulation as described herein, and one (or more) channels for providing a stimulation that causes, for example and without limitation, contraction of the EUS or the anal sphincter (e.g., stimulation at 0.5 Hz to 15 Hz) and/or contraction of the bladder or colon/rectum (e.g., 15 Hz to 50 Hz), such that the device can control micturition and/or defecation. Device **10, 110** can include multiple channels for providing a blocking stimulation as described herein, for example, and without limitation, to bilateral pudendal nerves (e.g., two branches of the pudendal nerve on contralateral sides of the body). A device **10, 110** useful for such treatment, e.g., a device having three output channels as described in U.S. Patent No. 9,623,243, can be used in a method including applying an electrical signal to a pudendal nerve or a branch thereof of a patient at a first point on the pudendal nerve or branch thereof, the electrical signal having an amplitude and frequency able to create a reflex that results in one or both of bladder contractions and rectal contractions; and applying a super-threshold electrical blocking signal (as described herein) distal to the first point on the pudendal nerve of the patient or a branch thereof and/or on a contralateral pudendal nerve or branch thereof, the blocking electrical signal having an amplitude and frequency able to block pudendal nerve conduction for inhibiting contraction of one or both of the external urethral sphincter and anal sphincter of the patient. The stopping and starting of the stimulation can be repeated multiple times to induce bladder or colon/rectal contraction when the pudendal nerves are blocked and the EUS and/or anal sphincter is relaxed, until the bladder or colon is fully emptied.

### Example 1

### Materials and Methods

Nine cats (5 males and 4 females; 3.3-4.2 kg; Marshall BioResources, North Rose, NY, USA) were anesthetized by isoflurane (2-5% oxygen) during surgery and switched to α-chloralose anesthesia (initial 65mg/kg i.v. with supplemental as needed) during data collection. The right cephalic vein was catheterized for administration of fluid or anesthetics. The airway was kept patent by a tracheotomy. A catheter was inserted into the right carotid artery to monitor the blood pressure. A pulse oximeter (9847V; NONIN Medical, Plymouth, MN) was attached to the tongue to monitor the heart rate and blood oxygen.

Via an abdominal incision a catheter was inserted into the distal urethra to slowly (1 ml/min) perfuse the urethra with saline and record urethral pressure increase caused by contractions of external urethral sphincter (EUS) that was induced by pudendal nerve stimulation **(****FIG. 3****).** The ureters were tied, cut, and drained externally. The left pudendal nerve was exposed via a 3-4 cm incision in the sciatic notch lateral to the tail for implantation of a tripolar cuff electrode (NEC113, MicroProbes Inc, Gaithersburg, MD, USA) to deliver HFBS (Stim.B in **FIG. 3****).** Two hook electrodes were placed central (Stim.C) and distal (Stim.D) to the tripolar cuff electrode. The right pudendal nerve was also exposed and implanted with the same 3 sets of electrodes. Pudendal nerves were transected centrally to prevent reflex activation of the EUS **(****FIG. 3****).** Stimulus pulses (20 Hz, 0.2 ms) generated by a stimulator (Grass S88, Grass Technologies, RI, USA) was delivered via a stimulation isolator (SIU5, Grass Technologies, RI, USA) to the hook electrodes (Stim.C or Stim.D) to induce >30 cmH₂O urethral pressure. LFBS (1kHz, 500 Hz, or 100 Hz) square waveform without a pulse interval, *see* **FIG. 3****)** generated by a computer running a LabView program (National Instrument, TX, USA) was delivered via a stimulation isolator (A395, World Precision Instruments, FL, USA) to the tripolar cuff electrode to block pudendal nerve conduction and suppress EUS contractions induced by Stim.C **(****FIG. 3****).** As initial nerve firing is not a concern when, for example, a patient has had a spinal cord injury, the 1 kHz LFBS can be applied at a super-threshold intensity (e.g., 5T, 10T, 20T, 30T, 40T, or 50T as shown in **FIGS. 4A-4B****)** that will significantly shorten the time to achieve a nerve conduction block.

### Results

**FIGS. 4A** **and** **4B** show that stimulation delivered at 1 kHz at 5T (0.5 mA) (here, T = excitation threshold of the nerve), 10T (1 mA), 20T (2 mA), 30T (3mA), 40T (mA), and 50T (5 mA) can produce nerve block and a significant post-stimulation block. **FIG. 5** shows that blocking can be accomplished at a lower frequency (500 Hz), when the intensity is 90T (9 mA) when the stimulation is delivered for 70 seconds, and **FIG. 6** shows that blocking can be accomplished at an even lower frequency (100 Hz) at an intensity of 90T (9 mA) when the stimulation is delivered for 30 seconds.

### Discussion

The results presented here show that LFBS, delivered at an intensity above the excitation threshold of a nerve or neuron (e.g., 5T, 10T, 20T, 30T, 40T, 50T), can quickly induce blocking and post-stimulation block in the nerve or neuron.

### Example 2

### Materials and Methods

### Experimental Protocol

A total of 10 cats (5 females and 5 males, 2.9-3.7 kg) were used in this study. The animals were anesthetized initially with isoflurane (2-5% in oxygen) during surgery and then switched to alpha-chloralose anesthesia (initial dose 65 mg/kg i.v. followed by supplemental doses as needed) during data collection. The right cephalic vein was catheterized for intravenous administration of fluid and drugs. A midline anterior cervical incision was used to access the airway, which was kept patent via tracheostomy. The right carotid artery was catheterized for monitoring arterial blood pressure. Oxygen saturation and heart rate were measured via a pulse oximeter (9847V, NONIN Medical, Plymouth, MN) attached to the tongue. Through an abdominal incision, the ureters were isolated, cut, and drained externally. A catheter was inserted into the urethra via a small incision in the proximal urethra. The catheter was connected to a syringe pump (SP200i; World Precision Instruments, Sarasota, FL) and a pressure transducer (BLPR2, World Precision Instruments) via a three-way stopcock to slowly (1 ml/min) perfuse the urethra and measure the urethral pressure increase caused by neutrally evoked contractions of the external urethral sphincter (EUS) **(****FIG. 3****).** Each pudendal nerve was exposed via a 3-4 cm incision in the sciatic notch lateral to the tail for implantation of a tripolar cuff electrode (NEC113, MicroProbes Inc, Gaithersburg, MD, USA) to deliver a biphasic stimulation waveform (Stim.B, FIG. 3). Bipolar hook electrodes were placed distal (Stim.D) and central (Stim.C) to the tripolar cuff electrode **(****FIG. 3****).** Each pudendal nerve was transected centrally to prevent reflex activation of the EUS. The nerve and electrodes were covered with warm (37 °C) mineral oil. Stimulus pulses (30 Hz, 0.2 ms) generated by a stimulator (Grass S88, Grass Technologies, RI, USA) were delivered via a stimulus isolator (SIU5, Grass Technologies, RI, USA) to the hook electrodes (Stim.C or Stim.D) to induce a EUS contraction and >30 cmH2O increase in urethral pressure. The biphasic stimulation waveform generated by a computer running a LabView program (National Instruments, TX, USA) was delivered via a stimulus isolator (A395, World Precision Instruments, FL, USA) to the tripolar cuff electrode to block pudendal nerve conduction and suppress EUS contractions (Fig. 1).

### Stimulation Protocol

The intensity threshold to block pudendal nerve conduction was determined at the beginning of every experiment by applying 10 kHz HFBS for 50-60 seconds at an increasing intensity starting from 1 mA with 1 mA increments. The minimal intensity for 10 kHz HFBS to completely suppress the urethral pressure induced by stimulation at Stim.C was determined as the block threshold (T) (see **FIG. 7****,** here T = the block threshold for 10 kHz stimulation to block nerve conduction). This frequency and duration of stimulation was shown in previous experiments to consistently block pudendal nerve conduction during the stimulation without causing a post-stimulation block. 10 kHz block threshold was chosen as the reference intensity in this study because 10 kHz nerve block is well known and recent studies show that 10 kHz requires a very long stimulation duration (10-30 minutes) to induce a post-stimulation block. In preliminary studies using the first 3 cats, LFBS (1 kHz, 500 Hz, or 100 Hz) was applied to the pudendal nerve at different intensities (1T, 2T or 3T) with different durations (5 secs to 3 minutes) randomly to determine if post-LFBS block could occur. In every experiment Stim.B was identified as the site of post-LFBS nerve block by showing that stimulation at the Stim.D site (30 Hz frequency, 0.2 ms pulse width, 5 secs duration) still induced >30 cmH2O EUS contractions when stimulation at the Stim.C site (30 Hz frequency, 0.2 ms pulse width, 5 secs on and 55 secs off) failed to induce EUS contractions (see **FIG. 8****).** In the remaining 7 cats, the minimal intensity and duration of LFBS (1 kHz, 500 Hz, or 100 Hz) to induce a complete post-LFBS block was examined in a systemic manner. A complete post-LFBS block was defined as the suppression of at least one of the EUS contractions induced by stimulation at Stim.C (30 Hz frequency, 0.2 ms pulse width, 5 secs on and 55 secs off) to <5 cmH2O urethral pressure. The testing was started using 1 kHz LFBS of 60 secs duration applied at an increasing intensity of 1T, 2T and 3T. If a complete post-LFBS block did not occur, then the LFBS intensity was kept at 3T and the stimulation duration was increased sequentially by 30 secs in repeated tests until a complete post-LFBS was observed (see **FIG. 9****).** This was then followed by 500 Hz LFBS at 1T starting at 5 secs duration. The duration was then increased sequentially in 5-10 sec increments to a maximum of 60 secs. If complete block was not elicited, the intensity was then increased to 2T at 60 sec and then to 3T if needed starting at 60 secs followed by sequential 5 second increases in duration until the complete post-LFBS block was observed. In the final series of tests, 100 Hz LFBS was initially applied at 1T intensity and 5 secs duration followed by sequential 5-10 sec increases to a maximum of 60 secs. If a complete post-LFBS block was not observed, the intensity was increased to 3T and 5 secs duration and then duration was increased sequentially in 5-10 secs steps until a complete post-LFBS block was observed. At the end of each LFBS test, a rest period (3-30 minutes) was inserted to allow the EUS contractions to fully recover before applying the next period of LFBS. 1 kHz was tested in 14 nerves (left and right in 7 cats), 500 Hz was tested in 9 nerves, and 100 Hz was tested in 6 nerves.

### Data Analysis

The recovery period was defined as the time period required for the EUS contraction pressure to reach >90% of the pre-stimulation pressure **(****FIG. 10****, top panel).** The minimal LFBS stimulation durations to induce a complete post-LFBS block at different frequencies (1 kHz, 500 Hz, and 100 Hz) were compared. The data obtained under the same conditions in different animals were averaged and presented as mean + standard error. Unpaired student t-test was performed to detect significant differences (p<0.05).

### Results

At the beginning of each experiment the urethral pressure responses indicating EUS contractions induced by electrical stimulation (30 Hz intermittent, 5 secs on and 55 secs off) of the pudendal nerve at sites central (Stim C) and distal (Stim D) to the blocking electrode (Stim B) **(****FIG. 3****)** were elicited over a range of intensities. Stimulus intensities (0.6-1.2 V, 0.2 ms pulse width) that produced approximately equal increases in urethral pressure >30 cmH2O at both sites were used for the remainder of the experiments to elicit control responses that were tested for sensitivity to LFBS block. Preliminary studies in the first 3 cats examined the effect of LFBS at different frequencies (1 kHz, 500 Hz, or 100 Hz) and stimulus intensities. Stimulus intensities were normalized in each animal by referencing to the stimulus intensity threshold (T) at 10 kHz that produced a transient increase in urethral pressure followed by a complete block of the urethral pressure response to stimulation at Stim.C (see **FIG. 7****).** As shown in **FIG. 8****,** the LFBS (Stim.B: 1 kHz, 3T = 9 mA, 120 secs) induced a tonic EUS contraction during the stimulation and a complete post-LFBS block that fully suppressed EUS contractions induced by electrical stimulation (30 Hz, 0.2 ms, 0.6 V, 5 secs on and 55 secs off) via the electrode at Stim.C. The EUS contractions gradually recovered in about 5.5 minutes following the LFBS **(****FIG. 8****).** During the post-LFBS block, stimulation at the distal electrode (Stim.D: 30 Hz, 0.2 ms. 0.6 V, 5 secs on) induced a EUS contraction of the same amplitude as that prior LFBS **(****FIG. 8****),** indicating that the LFBS blocked the pudendal nerve locally at the Stim.B site but not distally at sites in the nerve, the neuromuscular junction, or due to fatigue of the EUS muscle.

The search protocol to determine the minimal stimulation intensity and duration required to induce a complete post-LFBS block after 1 kHz stimulation is shown in **FIG. 9****.** As the 1 kHz LFBS of 60 secs duration increased in intensity from 1T to 3T, the post-LFBS block was first observed at 3T intensity as a 50% reduction in the EUS contraction pressure (see the top trace in **FIG. 9****).** At 3T intensity, further increasing the duration of stimulation from 60 secs to 150 secs produced a complete post-LFBS block (see the bottom trace in **FIG. 9****).** Therefore, the minimal stimulation intensity and duration were determined as 3T and 150 secs for 1 kHz LFBS to induce a complete nerve block **(****FIG. 9****).** Using the same nerve in this cat, the minimal stimulation intensity and duration for LFBS of 500 Hz **(****FIG. 10****, top panel)** or 100 Hz **(****FIG. 10****, bottom panel)** to induce a complete post-LFBS block were also determined. The recovery from complete block had a similar time course for both frequencies of stimulation **(****FIG. 10****).** Although 3T intensity was required in this cat for 1 kHz, 500 Hz, and 100 Hz LFBS to induce a complete post-LFBS block, the block was also observed at 1T or 2T intensity in the other 6 cats at different frequencies. On average (N=7 cats) the minimal stimulation duration to induce a complete block significantly (p<0.05) increased from 23±8 secs to 95±14 secs when the frequency increased from 100 Hz to 1 kHz **(****FIG. 11****, top panel)** although the block occurred at various stimulation intensities (1-3T). At the minimal stimulation intensity and duration, the complete post-LFBS block induced by stimulation of different frequencies had similar complete block duration **(****FIG. 11****, middle panel)** and recovery period **(****FIG. 11****, lower panel).** The post-LFBS block induced at the minimal stimulation intensity and duration was fully reversible **(****FIGS. 8-10****)** in every experiment within the same time period (10-15 minutes on average) for the three frequencies **(****FIG. 11****, middle and bottom panels).**

### Discussion

This study in cats discovered that post-stimulation block of pudendal nerve conduction could be induced by LFBS at a frequency less than 1 kHz **(****FIGS. 8-10****)** and at a lower frequency, a shorter stimulation duration is required to induce a complete post-LFBS block **(****FIG. 11****, top panel).** The block induced by LFBS at the minimal intensity and duration is fully reversible within a similar recovery period for different frequencies **(****FIG. 11****, middle and bottom panels).** These results provide valuable information for understanding the possible mechanisms underlying the block of nerve conduction by biphasic stimulation waveforms. This study also opens the opportunity for clinical applications to induce post-stimulation block at a low frequency (≤1 kHz) within 30-60 seconds **(****FIG. 11****, top panel)** while the traditional 10 kHz HFBS requires a much longer stimulation duration (10-30 minutes) to achieve a post-stimulation block.

The post-LFBS block is fully reversible, which indicates that the nerve is likely not damaged by the LFBS. Therefore, it is reasonable to assume that the post-LFBS block is caused by alteration of the ionic mechanisms underlying axonal conduction. Previous computer simulation studies revealed that each stimulus pulse of the biphasic stimulation waveform can generate an inward sodium current and an outward potassium current. Therefore, it is reasonable to expect that the intracellular and extracellular ion concentrations must be changed dramatically as the LFBS continues. When LFBS is terminated, these large changes in ion concentrations must disrupt the normal transmembrane ionic gradients necessary for the generation of the action potential and cause nerve conduction block. As the transmembrane ion pumps gradually restore the normal intracellular and extracellular ion concentrations, the post-LFBS block will slowly disappear and the nerve conduction will be restored. This mechanism revealed by computer simulation studies can explain very well the post-LFBS block observed in current study. It is worth noting that the LFBS induced a tonic EUS contraction during the stimulation but blocked the nerve conduction after the stimulation **(****FIGS. 8-10****).** This is in dramatic contrast to the HFBS (10 kHz) that instead of inducing a tonic EUS contraction produced a nerve block during the stimulation but did not block the nerve after the stimulation **(****FIG. 7****).** These results indicate that the mechanisms are very different for post-LFBS block and the block induced by HFBS during the stimulation. In fact, our previous computer simulation studies did reveal a different blocking mechanism for HFBS that maintains potassium channels in a constantly open state at stimulation frequencies ≥5 kHz. This causes nerve conduction block during stimulation **(****FIG. 7****)** instead of the block that occurs after stimulation that is mediated by changes in transmembrane ionic gradients.

It is also important to note that at a lower frequency each stimulus pulse of the biphasic stimulation waveform (see **FIG. 3****)** has a longer duration that can drive more sodium and potassium ions across the axonal membranes and therefore be more effective in changing the ion concentrations than higher frequencies of stimulation that have a shorter duration stimulus pulse. This may explain why the lower frequency (100 Hz) stimulation requires a shorter stimulation duration than the higher frequencies (500 Hz or 1 kHz) to induce a complete post-LFBS block **(****FIG. 11****, top panel).** This finding also raises the question of whether a lower frequency (<100 Hz) would be effective at an even shorter duration? However, the proposed mechanism must have a minimal effective frequency because during a very long stimulus pulse, the sodium and potassium channels will open at the beginning of the pulse but will be closed before the end of the pulse due to the ion channel kinetics. Therefore, for a very low frequency (i.e., a very long stimulus pulse) the LFBS will become less effective as the inward sodium and outward potassium currents during the last phase of the long stimulus pulse will decline and have less impact on the intracellular and extracellular ion concentrations.

Although the minimal stimulation duration for producing post-LFBS block is frequency dependent, the nerve blocks induced by different LFBS frequencies have a similar time course for recovery **(****FIG. 11****, middle and bottom panels).** This is expected because complete conduction block at different frequencies is probably occurring after the same change in ion concentrations induced by LFBS at the minimal intensity and duration; whereas recovery occurs after a time required for the membrane pumps to restore normal ion concentrations, which should be frequency independent because the starting point for recovery should be the same after 100 Hz to 1 kHz stimulation However, based on the ion concentration hypothesis the duration of post-LFBS block should be increased by LFBS of a high intensity or a long duration. The maximal duration of post-LFBS block induced by a strong LFBS of a long duration without causing nerve tissue damage still needs to be determined. In addition, the intensity-duration relationship for LFBS at a fixed frequency to induce a post-LFBS block also needs to be studied as well as the intensity frequency relationship for LFBS of a fixed duration. These relationships will provide more information to support or refute the ion concentration hypothesis of nerve block.

The LFBS always produces tonic nerve firing during stimulation before it can induce a post-LFBS block **(****FIGS. 8-10****).** Therefore, it is less likely that LFBS will be used clinically to block peripheral nerves for treatment of chronic pain. However, the post-LFBS block will be very useful in clinical applications where initial tonic nerve firing is acceptable. For example, post-LFBS block can be used to prevent detrusor sphincter dyssynergia and allow efficient voiding after spinal cord injury by blocking the pudendal nerve conduction as shown in this study. During the 2-5 minutes of complete post-LFBF pudendal nerve block **(****FIG. 11****, middle panel),** the EUS relaxes to allow efficient voiding to occur at a low bladder pressure.

In summary, this study discovered a novel method to block nerve conduction using a biphasic stimulation waveform of a low (≤1 kHz) frequency. The results confirmed that post-stimulation block can be induced not only by HFBS (≥5kHz) but also by LFBS (≤1 kHz), supporting the theory that ion concentration changes may play an important role in nerve conduction block by biphasic stimulation waveform. The post-LFBS block could be used in many clinical applications where initial tonic nerve firing is acceptable, providing opportunities to develop new neuromodulation devices.

### Example 3

### MATERIALS AND METHODS

The experiment setup as shown in **FIG. 3** was also used to obtain the results shown in **FIG 12****.** However, the cuff electrode (Stim.B in **FIG. 3****)** was replaced by a hook electrode or by a single lead electrode to deliver LFBS to block the pudendal nerve conduction.

### RESULTS

As shown in **FIG. 12****, top panel** a bipolar hook electrode, delivering a blocking stimulation (Stim. B) at 1 kHz, 0.5 mA, 0.2 ms pulsewidth for 2.5 minutes can provide post-stimulation blocking. As also shown **(****FIG. 12****, bottom panel),** use of a single lead electrode to deliver a LFBS (100 Hz, 0.5 mA, 0.2 ms pulsewidth, for 30 seconds) can similarly provide post-stimulation blocking.

### DISCUSSION

The results presented here show that a bipolar/tripolar cuff electrode, which is highly invasive in terms of needing access in order to surround, or at least partially surround, a nerve is not required to deliver LFBS. Rather, a simple electrode lead, placed in proximity to a nerve of interest (e.g., the pudendal nerve), can provide blocking, with post-stimulation blocking, to deliver the therapeutic effects discussed herein. The present invention is set out in the claims that follow.

## Claims

1. A device (110) for controlling micturition or defecation in a patient comprising:
a controller (110);
one or two pulse generators (120) in communication with the controller; and
one or more lead (130, 135) and/or cuff electrodes, configured to be placed near or in contact with the left and/or right pudendal nerves, the electrodes in electrical communication with the first pulse generator, wherein
the device is configured to apply an electrical stimulation to the pudendal nerve, wherein the electrical stimulation is of an intensity that is equal to or greater than an excitation threshold of the pudendal nerve for a length of time sufficient to produce a block of pudendal nerve conduction either during or after ending the electrical stimulation, thereby inhibiting contraction of the external urethral sphincter (EUS) and/or the external anal sphincter (EAS) wherein the electrical stimulation for pudendal nerve block is delivered at a frequency of 1 Hz to less than 4 kHz; and, either
another electrode, configured to be placed near or in contact with the blocked pudendal nerve at a site central to the blocked site, or be placed near or in contact with the sacral (S1, S2, S3, or S4) spinal root, spinal cord, or pelvic nerve,
the another electrode in electrical communication with the first pulse generator, wherein
the device is configured to apply electrical stimulation using the electrode either during or immediately after the ending of pudendal nerve blocking stimulation at an intensity that is greater than an excitation threshold of the pudendal nerve, sacral (S1, S2, S3, or S4) spinal root, spinal cord, or pelvic nerve to induce bladder or colon/rectal contraction; or
one or more skin surface electrodes or electromagnetic coils, configured to be placed on the skin surface along the patient's spine and in electrical communication with the second pulse generator, and wherein
the device is configured to apply electrical stimulation using the skin surface electrodes or electromagnetic coils either during or immediately after the ending of pudendal nerve blocking stimulation at an intensity that is greater than an excitation threshold of the spinal cord to induce bladder or colon/rectal contraction.

2. The device of claim 1, wherein the electrical stimulation for pudendal nerve block is delivered at an intensity that is at least five times the excitation threshold of the pudendal nerve.

3. The device of claim 1, wherein the electrical stimulation for pudendal nerve block is delivered at a frequency of 1 Hz to 1.5 kHz.

4. The device of claim 1, wherein the electrical stimulation for pudendal nerve block is delivered for a period of from 5 seconds to 60 minutes.

5. The device of claim 1, wherein the electrical stimulation results in the block of pudendal nerve conduction for at least 10 seconds following cessation of the electrical stimulation.

6. The device of claim 1, wherein the electrical stimulation for pudendal nerve block comprises biphasic electrical pulses.

7. The device of claim 6, wherein the biphasic pulses are symmetric between the positive and negative phases of the biphasic pulse.

8. The device of claim 6, wherein the biphasic pulses are asymmetric between the positive and negative phases of the biphasic pulse.

9. The device of claim 1, wherein the electrical stimulation comprises biphasic electrical pulses that are charge-balanced.

10. The device of claim 1, wherein the device is further configured to once block of pudendal nerve conduction is achieved, stop application of the pudendal nerve blocking electrical stimulation for a period of at least 10 seconds, wherein the block of pudendal nerve conduction is maintained during the period and, after the period has concluded, resume the electrical stimulation of the pudendal nerve or branch thereof at the same or different intensity and/or at the same or different frequency to continue or prolong the block of pudendal nerve conduction.

11. The device of claim 1, wherein the device is further configured to once block of pudendal nerve conduction is achieved, maintain the block by changing the intensity and/or frequency of the pudendal nerve blocking electrical stimulation.

12. The device of claim 1, wherein the electrical stimulation for inducing bladder or colon/rectal contraction and applied to the pudendal nerve, the sacral (S1, S2, S3, or S4) spinal root, spinal cord, or the pelvic nerve has a frequency between 1 Hz to 100 Hz, optionally between 15 Hz and 50 Hz, or optionally between 1 Hz to 3 Hz.

13. The device of claim 12, wherein the stimulation is delivered at an intensity of 0.01 mA to 20 mA and/or 1 mV to 20 V, optionally 0.1 mA to 10 mA.

14. The device of claim 12, wherein the device is further configured to deliver the stimulation for a period longer than the period of bladder or colon/rectal contraction.

15. The device of claim 12, wherein the device is further configured to, once the bladder or colon/rectal contraction has ended, stop application of the electrical stimulation for a period of at least 10 seconds after the period has concluded, resume the stimulation at the same or different intensity and/or at the same or different frequency to induce bladder or colon/rectal contraction again while the pudendal nerves are blocked and the EUS/EAS is relaxed.

## Patentansprüche

1. Vorrichtung (110) zur Steuerung des Wasserlassens oder Stuhlgangs bei einem Patienten, umfassend:
eine Steuereinheit (110);
einen oder zwei Impulsgeneratoren (120), die mit der Steuereinheit in Verbindung stehen; und
eine oder mehrere Elektroden (130, 135) in Form von Leitungen und/oder Manschetten, die so ausgestaltet sind, dass sie in der Nähe des linken und/oder rechten Pudendusnervs oder in Kontakt mit diesem platziert werden können, wobei die Elektroden in elektrischer Verbindung mit dem ersten Impulsgenerator stehen, wobei
die Vorrichtung so ausgestaltet ist, dass sie eine elektrische Stimulation an den Pudendusnerv abgibt, wobei die elektrische Stimulation eine Intensität aufweist, die gleich oder größer als eine Erregungsschwelle des Pudendusnervs ist, und zwar über einen Zeitraum, der ausreicht, um entweder während oder nach Beendigung der elektrischen Stimulation eine Blockade der Pudendusnervenleitung zu bewirken, wodurch die Kontraktion des äußeren Harnröhrenschließmuskels (EUS) und/oder des äußeren Analsphinkters (EAS) gehemmt wird, wobei die elektrische Stimulation zur Blockade des Pudendusnervs mit einer Frequenz von 1 Hz bis weniger als 4 kHz abgegeben wird; und entweder
eine weitere Elektrode, die so ausgestaltet ist, dass sie in der Nähe des blockierten Pudendusnervs oder in Kontakt mit diesem an einer Stelle zentral zur Blockadestelle platziert wird, oder in der Nähe der sakralen (S1, S2, S3 oder S4) Spinalwurzel, des Rückenmarks oder eines Beckennervs platziert wird,
wobei die weitere Elektrode in elektrischer Verbindung mit dem ersten Impulsgenerator steht, wobei
die Vorrichtung so ausgestaltet ist, dass sie mithilfe der Elektrode entweder während oder unmittelbar nach Beendigung der Stimulation zur Blockade des Pudendusnervs eine elektrische Stimulation mit einer Intensität anwendet, die größer ist als eine Erregungsschwelle des Pudendusnervs, der sakralen (S1, S2, S3 oder S4) Spinalwurzel, des Rückenmarks oder des Beckennervs, um eine Kontraktion der Blase oder des Dickdarms/Enddarms auszulösen; oder
eine oder mehrere Hautoberflächenelektroden oder elektromagnetische Spulen so ausgestaltet sind, dass sie auf der Hautoberfläche entlang der Wirbelsäule des Patienten platziert werden können und in elektrischer Verbindung mit dem zweiten Impulsgenerator stehen, und wobei
die Vorrichtung so ausgestaltet ist, dass sie mithilfe der Hautoberflächenelektroden oder elektromagnetischen Spulen entweder während oder unmittelbar nach Beendigung der Stimulation zur Blockade des Pudendusnervs eine elektrische Stimulation mit einer Intensität anwendet, die größer ist als die Erregungsschwelle des Rückenmarks, um eine Kontraktion der Blase oder des Dickdarms/Enddarms auszulösen.

2. Vorrichtung gemäß Anspruch 1, wobei die elektrische Stimulation zur Blockade des Pudendusnervs mit einer Intensität abgegeben wird, die mindestens das Fünffache der Erregungsschwelle des Pudendusnervs beträgt.

3. Vorrichtung gemäß Anspruch 1, wobei die elektrische Stimulation zur Blockade des Pudendusnervs mit einer Frequenz von 1 Hz bis 1,5 kHz abgegeben wird.

4. Vorrichtung gemäß Anspruch 1, wobei die elektrische Stimulation zur Blockade des Pudendusnervs über einen Zeitraum von 5 Sekunden bis 60 Minuten abgegeben wird.

5. Vorrichtung gemäß Anspruch 1, wobei die elektrische Stimulation zur Blockade der Pudendusnervenleitung für mindestens 10 Sekunden nach Beendigung der elektrischen Stimulation führt.

6. Vorrichtung gemäß Anspruch 1, wobei die elektrische Stimulation zur Blockade des Pudendusnervs zweiphasige elektrische Impulse umfasst.

7. Vorrichtung gemäß Anspruch 6, wobei die zweiphasigen Impulse zwischen der positiven und der negativen Phase des zweiphasigen Impulses symmetrisch sind.

8. Vorrichtung gemäß Anspruch 6, wobei die zweiphasigen Impulse zwischen der positiven und der negativen Phase des zweiphasigen Impulses asymmetrisch sind.

9. Vorrichtung gemäß Anspruch 1, wobei die elektrische Stimulation aus zweiphasigen elektrischen Impulsen besteht, die ladungsausgeglichen sind.

10. Vorrichtung gemäß Anspruch 1, wobei die Vorrichtung ferner so ausgestaltet ist, dass sie, sobald eine Blockade der Pudendusnervenleitung erreicht ist, die Anwendung der den Pudendusnerv blockierenden elektrischen Stimulation für einen Zeitraum von mindestens 10 Sekunden unterbricht, wobei die Blockade der Pudendusnervenleitung während dieses Zeitraums aufrechterhalten wird, und sie nach Ablauf dieses Zeitraums die elektrische Stimulation des Pudendusnervs oder eines seiner Äste mit derselben oder einer anderen Intensität und/oder mit derselben oder einer anderen Frequenz wieder aufnimmt, um die Blockade der Pudendusnervenleitung fortzusetzen oder zu verlängern.

11. Vorrichtung gemäß Anspruch 1, wobei die Vorrichtung ferner so ausgestaltet ist, dass sie, sobald eine Blockade der Pudendusnervenleitung erreicht ist, die Blockade aufrechterhält, indem sie die Intensität und/oder Frequenz der elektrischen Stimulation zur Blockade des Pudendusnervs verändert.

12. Vorrichtung gemäß Anspruch 1, wobei die elektrische Stimulation zur Auslösung einer Blasen- oder Kolon-/Rektalkontraktion, die an den Pudendusnerv, die sakrale (S1, S2, S3 oder S4) Spinalwurzel, das Rückenmark oder den Beckennerv angelegt wird, eine Frequenz zwischen 1 Hz und 100 Hz, gegebenenfalls zwischen 15 Hz und 50 Hz oder gegebenenfalls zwischen 1 Hz und 3 Hz aufweist.

13. Vorrichtung gemäß Anspruch 12, wobei die Stimulation mit einer Intensität von 0,01 mA bis 20 mA und/oder 1 mV bis 20 V, gegebenenfalls 0,1 mA bis 10 mA, abgegeben wird.

14. Vorrichtung gemäß Anspruch 12, wobei die Vorrichtung ferner so ausgestaltet ist, dass sie die Stimulation über einen Zeitraum abgibt, der länger ist als die Dauer der Blasen- oder Dickdarm-/Rektalkontraktion.

15. Vorrichtung gemäß Anspruch 12, wobei die Vorrichtung ferner so ausgestaltet ist, dass sie, sobald die Blasenoder Kolon-/Rektalkontraktion beendet ist, die Abgabe der elektrischen Stimulation für einen Zeitraum von mindestens 10 Sekunden nach Beendigung der Kontraktion unterbricht und die Stimulation mit derselben oder einer anderen Intensität und/oder mit derselben oder einer anderen Frequenz wieder aufnimmt, um erneut eine Blasenoder Kolon-/Rektalkontraktion auszulösen, während die Pudendusnerven blockiert sind und der EUS/EAS entspannt ist.

## Revendications

1. Dispositif (110) destiné à contrôler la miction ou la défécation chez un patient, comprenant :
un contrôleur (110) ;
un ou deux générateurs d'impulsions (120) en communication avec le contrôleur ; et
une ou plusieurs électrodes de sonde (130, 135) et/ou électrodes à manchon, configurées pour être placées à proximité ou en contact avec les nerfs pudendaux gauche et/ou droit, lesdites électrodes étant en communication électrique avec le premier générateur d'impulsions, dans lequel
le dispositif est configuré pour appliquer une stimulation électrique au nerf pudendal, ladite stimulation électrique ayant une intensité égale ou supérieure à un seuil d'excitation du nerf pudendal pendant une durée suffisante pour produire un blocage de la conduction du nerf pudendal soit pendant, soit après la fin de la stimulation électrique, inhibant ainsi la contraction du sphincter urétral externe (EUS) et/ou du sphincter anal externe (EAS), la stimulation électrique destinée au blocage du nerf pudendal étant délivrée à une fréquence comprise entre 1 Hz et moins de 4 kHz ; et, soit
une autre électrode, configurée pour être placée à proximité ou en contact avec le nerf pudendal bloqué, à un emplacement central par rapport au site de blocage, soit pour être placée à proximité ou en contact avec la racine spinale sacrée (S1, S2, S3 ou S4), la moelle épinière ou un nerf pelvien,
cette autre électrode étant en communication électrique avec le premier générateur d'impulsions, dans lequel
le dispositif est configuré pour appliquer une stimulation électrique à l'aide de l'électrode soit pendant, soit immédiatement après la fin de la stimulation de blocage du nerf pudendal, à une intensité supérieure au seuil d'excitation du nerf pudendal, de la racine spinale sacrée (S1, S2, S3 ou S4), de la moelle épinière ou du nerf pelvien, afin de provoquer une contraction de la vessie ou du côlon/rectum ; ou
une ou plusieurs électrodes de surface cutanée ou bobines électromagnétiques, configurées pour être placées à la surface de la peau le long de la colonne vertébrale du patient et en communication électrique avec le deuxième générateur d'impulsions, et dans lequel
le dispositif est configuré pour appliquer une stimulation électrique à l'aide des électrodes de surface cutanée ou des bobines électromagnétiques soit pendant, soit immédiatement après la fin de la stimulation de blocage du nerf pudendal, à une intensité supérieure au seuil d'excitation de la moelle épinière, afin de provoquer une contraction de la vessie ou du côlon/rectum.

2. Dispositif selon la revendication 1, dans lequel la stimulation électrique destinée au blocage du nerf pudendal est délivrée à une intensité au moins cinq fois supérieure au seuil d'excitation du nerf pudendal.

3. Dispositif selon la revendication 1, dans lequel la stimulation électrique destinée au blocage du nerf pudendal est délivrée à une fréquence comprise entre 1 Hz et 1,5 kHz.

4. Dispositif selon la revendication 1, dans lequel la stimulation électrique destinée au blocage du nerf pudendal est délivrée pendant une durée comprise entre 5 secondes et 60 minutes.

5. Dispositif selon la revendication 1, dans lequel la stimulation électrique entraîne le blocage de la conduction du nerf pudendal pendant au moins 10 secondes après l'arrêt de la stimulation électrique.

6. Dispositif selon la revendication 1, dans lequel la stimulation électrique destinée au blocage du nerf pudendal comprend des impulsions électriques biphasiques.

7. Dispositif selon la revendication 6, dans lequel les impulsions biphasiques sont symétriques entre les phases positive et négative de l'impulsion biphasique.

8. Dispositif selon la revendication 6, dans lequel les impulsions biphasiques sont asymétriques entre les phases positive et négative de l'impulsion biphasique.

9. Dispositif selon la revendication 1, dans lequel la stimulation électrique comprend des impulsions électriques biphasiques à charge équilibrée.

10. Dispositif selon la revendication 1, dans lequel le dispositif est en outre configuré pour, une fois le blocage de la conduction du nerf pudendal obtenu, d'interrompre l'application de la stimulation électrique bloquant le nerf pudendal pendant une durée d'au moins 10 secondes, le blocage de la conduction du nerf pudendal étant maintenu pendant cette durée, et, une fois cette durée écoulée, de reprendre la stimulation électrique du nerf pudendal ou d'une de ses branches à une intensité identique ou différente et/ou à une fréquence identique ou différente afin de poursuivre ou de prolonger le blocage de la conduction du nerf pudendal.

11. Dispositif selon la revendication 1, dans lequel le dispositif est en outre configuré pour, une fois le blocage de la conduction du nerf pudendal obtenu, maintenir ce blocage en modifiant l'intensité et/ou la fréquence de la stimulation électrique bloquant le nerf pudendal.

12. Dispositif selon la revendication 1, dans lequel la stimulation électrique destinée à induire une contraction de la vessie ou du côlon/rectum et appliquée au nerf pudendal, à la racine spinale sacrée (S1, S2, S3 ou S4), à la moelle épinière ou au nerf pelvien a une fréquence comprise entre 1 Hz et 100 Hz, éventuellement entre 15 Hz et 50 Hz, ou éventuellement entre 1 Hz et 3 Hz.

13. Dispositif selon la revendication 12, dans lequel la stimulation est délivrée à une intensité comprise entre 0,01 mA et 20 mA et/ou entre 1 mV et 20 V, éventuellement entre 0,1 mA et 10 mA.

14. Dispositif selon la revendication 12, dans lequel le dispositif est en outre configuré pour délivrer la stimulation pendant une durée supérieure à la durée de la contraction de la vessie ou du côlon/rectum.

15. Dispositif selon la revendication 12, dans lequel le dispositif est en outre configuré pour, une fois que la contraction de la vessie ou du côlon/rectum a pris fin, interrompre l'application de la stimulation électrique pendant une durée d'au moins 10 secondes après la fin de ladite contraction, puis reprendre la stimulation à une intensité identique ou différente et/ou à une fréquence identique ou différente afin de provoquer à nouveau une contraction de la vessie ou du côlon/rectum tandis que les nerfs pudendaux sont bloqués et que l'EUS/EAS est détendu.
